# EUROPEAN PATENT APPLICATION

(11) **EP 4 837 533 A1**
(43) Date of publication of application: **11.11.2026**
(21) Application number: 25180578.4
(22) Date of filing: 03.06.2025
(51) Int. Cl.: A61B 18/18

(54) **SKIN TREATMENT DEVICE AND SKIN TREATMENT SYSTEM**

(30) Priority: 09.05.2025 US 202563802821 P
(71) Applicant: Braun GmbH, 65824 Schwalbach am Taunus (DE)
(72) Inventor: KAMYSHANSKA, Hanna, 61476 Kronberg (DE); PATTNAIK, Shrikant Prasad, Boston, 02127-1096 (US); LI, Pei, 61476 Kronberg (DE); DIETRICH, Franziska, 61476 Kronberg (DE); PENA, Humberto, 61476 Kronberg (DE); WANG, Sandy, Boston, 02127-1096 (US)
(74) Representative: P&G Patent Germany

(57) **Abstract**

The present application is concerned with skin treatment system having a skin treatment device and a separate interaction device, the separate interaction device and the skin treatment device being arranged for at least one-directional communication from the skin treatment device to the separate interaction device, the separate interaction device having at least one indicator element for providing user-perceivable information, the skin treatment system having a first usage mode, at least a second usage mode and a processor, and wherein the processor is arranged to provide a higher level of feedback and/or guidance and/or user interaction in the second usage mode via the at least one indicator element during a treatment session of the skin treatment device than in the first usage mode.

## Description

### FIELD OF THE INVENTION

The present invention is concerned with a skin treatment device or skin treatment system that provides improved user friendliness. The skin treatment device or skin treatment system comprises a processor and at least one indicator element for providing user-perceivable information.

### BACKGROUND OF THE INVENTION

Skin treatment devices are generally known. It is also known to combine a skin treatment system with a separate interaction device to form a skin treatment system. E.g., the Braun skin i·expert IPL ("Intense Pulsed Light") system comprises an IPL device and a user-owned smartphone on which an App can be downloaded to support the user when utilizing the device.

There is a general need to improve a skin treatment device or skin treatment system so that specifically a new user may be better guided to understand and/or utilize the skin treatment device / system.

### SUMMARY OF THE INVENTION

In accordance with at least one aspect, a skin treatment device is provided that comprises a user interface including at least one indicator element for providing user perceivable information, a first usage mode, and at least a second usage mode, a processor, wherein the processor is arranged to provide a higher level of feedback and/or guidance and/or user interaction in the second usage mode via the at least one indicator element during a treatment session of the skin treatment device than in the first usage mode.

In accordance with at least one aspect, a skin treatment system is provided that comprises a skin treatment device, specifically a skin treatment device in accordance with the previous paragraph, and a separate interaction device, the separate interaction device and the skin treatment device being arranged for at least one-directional communication from the skin treatment device to the separate interaction device, in particular for bi-directional communication, further in particular wherein the communication is a wireless communication, the separate interaction device comprising at least one indicator element for providing user-perceivable information, the skin treatment system comprising a first usage mode, at least a second usage mode and a processor, and wherein the processor is arranged to provide a higher level of user feedback and/or guidance and/or user interaction in the second usage mode via the at least one indicator element during a usage session of the skin treatment device than in the first usage mode.

### BRIEF DESCRIPTION OF THE DRAWINGS

The present disclosure will be further elucidated by a detailed description of example embodiments and with reference to figures. In the figures
- Fig. 1: is a schematic depiction of an example skin treatment device;
- Fig. 2: is a schematic depiction of an example skin treatment system comprising a skin treatment device and a separate interaction device;
- Fig. 3A: is a depiction of a first example information and/or input screen from a first sequence of three information and/or input screens that show potential usage guidance (UG) / interactive usage guidance (IUG) / personalized usage guidance (PUG) teaching the user the functions of the skin treatment device;
- Fig. 3B: is a depiction of the second information and/or input screen from the first sequence of three information and/or input screens;
- Fig. 3C: is a depiction of the third information and/or input screen from the first sequence of three information and/or input screens;
- Fig. 4A: is a depiction of a first example information and/or input screen from a second sequence of three information and/or input screens that show potential usage guidance (UG) / interactive usage guidance (IUG) / personalized usage guidance (PUG) teaching the user the functions of the skin treatment device;
- Fig. 4B: is a depiction of the second information and/or input screen from the second sequence of three information and/or input screens;
- Fig. 4C: is a depiction of the third information and/or input screen from the second sequence of three information and/or input screens; and
- Fig. 5: is a depiction of an example input screen that may be used in a personalized usage guidance to allow a user to input personalized data.

### DETAILED DESCRIPTION OF THE INVENTION

A "skin treatment device" may treat skin with light or with other means, such as mechanical treatment elements (e.g., pincers for ripping out hairs, or filaments, tufts or other nurturing elements for brushing and/or massaging the skin) or electric treatment elements (e.g., electrodes for applying a treatment current). A skin treatment device may provide treatments that serve a purely cosmetic effect (such as shaving or other types of hair removal or hair growth management) or a purely therapeutic or prophylactic therapeutic effect (such as application of blue light for reduction of medical skin issues) or treatments where cosmetic and therapeutic effects are combined. In the following, the description mainly focuses on the application of intense light pulses for the purpose of cosmetic hair growth management / cosmetic hair growth reduction. Such skin treatment devices are commonly named IPL (Intense Pulsed Light) devices.

A skin treatment device may comprise at least one treatment light source that is housed in a light chamber, where the term light chamber refers to a hollow surrounded by at least one (continuous) wall, which at least one wall may be reflective for the treatment light, i.e. the wall may be made from metal that may be polished on its inner wall surface ("inner" here refers to the wall surface that faces the treatment light source) or may comprise a polished or generally mirror-like coating such as a metal coating. Instead of a single continuous wall that defines the inner hollow of the light chamber, several walls may be combined to define the hollow of the light chamber. The light chamber typically has one opening via which the treatment light emitted by the at least one treatment light source can exit the light chamber and can be applied, e.g., onto a subject's skin, which opening is also called "light exit window". Here, "exit" shall refer to one feature of the light exit window, namely to allow treatment light to exit the light chamber but of course light, in particular treatment light backscattered or reflected by the skin or ambient light also can "enter" the light chamber via the light exit window. The light exit window defines a treatment area that may have a size in a range of between 0.1 mm² to 25 cm², in particular in a range of between 0.25 cm² to 10 cm², further in particular in a range of between 0.5 cm² to 5 cm², such as 1.0 cm², 1.5 cm², 2.0 cm², 2.5 cm², 3.0 cm², 3.5 cm², 4.0 cm², or 4.5 cm².

A skin treatment system in accordance with the present application comprises a skin treatment device and a separate interaction device. The separate interaction device may be a proprietary device that may be made by the same manufacturer, and the two devices may be sold in combination or individually. It is also common that a separate interaction device is realized by a typically already user-owned smart device such as a cell phone, smartphone, tablet, AR/VR glasses, smart glasses or the like and a specific App may be offered in combination to program the user-owned smart device in such a way that it realizes the features of the separate interaction device. User-owned smart devices may readily be provided with a processor, a (touch sensitive) display, a loudspeaker, a vibrator, a camera and/or a transceiver for establishing a wireless communication

The focus of the present proposed skin treatment device or skin treatment system lies in the provision of two different usage modes that differ in a pre-defined level of guidance given to a user of the skin treatment device or skin treatment system, a level of feedback, specifically a level of feedback provided during an ongoing treatment session, given to a user of the skin treatment device or skin treatment system, and/or a level of user interaction that the skin treatment device or the skin treatment system demands from the user prior to and/or during an ongoing treatment session. In this context, "guidance" and "feedback" are considered to be "non-interactive" as they do not require any user interaction, while "user interaction" requires certain tasks such device actions to be performed or information to be inputted while "guidance" or "feedback" is provided. The respective guidance or feedback may then pause until the user interaction has completed (or the user deactivates the user interaction portion). The "guidance", "feedback" and the "user interaction" may become personalized by taking into account personalized data. While it is always considered that a user has the freedom to deactivate certain aspects of the guidance or feedback or user interaction provided in the second usage mode, it is to be understood that the respective start levels are predefined and thus set the scope of the second usage mode.

In the following, the terms "General Guidance", "Usage Guidance", "Interactive Usage Guidance", "Personalized Usage Guidance", "Feedback on Usage" and "Feedback on Results Achieved" are explained as examples relating to the mentioned guidance, feedback and user interaction provided by the skin treatment device / skin treatment system.

### General Guidance (GG)

- Is intended to improve the user's understanding of the treatment as such and any body parts being treated in the course of a treatment session.
- Is intended to automatically be provided at a start of a treatment session and/or during the treatment session in a second usage mode, e.g., directly after the skin treatment device or skin treatment system is switched on or when the user pushes a button of the skin treatment device or skin treatment system.
- Individual elements of the GG may additionally be "off-line" available, e.g., the user can manually select individual GG aspects such as a video or general information etc.
- All or individual parts of the GG may be de-selectable by a user to shape a user-defined second usage mode.

### Usage Guidance (UG)

- Is intended to improve the user's understanding and handling of the skin treatment device or skin treatment system.
- Is intended to automatically be provided at the start of the treatment session and/or during the treatment session in the second usage mode, e.g., directly after the skin treatment device or skin treatment system is switched on or when the user pushes a button of the skin treatment device or skin treatment system.
- Individual elements of the UG may be "off-line" available.
- All or individual parts of the UG may be de-selectable by a user to shape a user-defined second usage mode.

### Interactive Usage Guidance (IUG)

- Is intended to improve the user's understanding and handling of the skin treatment device or skin treatment system; the interactive nature tends to improve that the learning indeed reaches the user.
- Is intended to automatically be provided in the second usage mode; a level of the IUG may be adjustable and the user may decide to selectively reduce the level or deactivate individual elements of the IUG or the complete IUG.
- In the context of IUG, "interactive" means that the user is asked to perform a certain task (e.g., push a button in a described manner or move the treatment head over the skin or to bring the treatment head into contact with the skin) or to input certain information.

### Personalized Usage Guidance (PUG)

- The PUG may be a part of UG/IPG.
- Personal data / information utilized in personalizing guidance or feedback may comprise at least one of body height, weight, sex, age, BMI - body-mass index, experience level, and ethnicity.
- The user may decide to deactivate the personalization of the provided guidance or feedback; the skin treatment device or skin treatment system may then use pre-defined standard values instead of personalized values.

### Feedback on Usage (FU)

- The FU is intended to improve the user's understanding and handling of the skin treatment device or skin treatment system.
- The FU is intended to automatically be provided in the second usage mode; the level may be adjustable by the user; the user may decide to selectively deactivate and activate individual aspects of the FU.
- The FU may depend on personalized data / information so that the feedback becomes "personalized feedback on usage" (PFU) as similarly indicated under PUG above.

### Feedback on Results Achieved (FRA)

- The FRA is intended to make the user understand the results that were achieved in a treatment session or with a sub-treatment action, e.g., with a single stroke the user made with the device; the FRA may indicate the changes in the results that can be achieved when changing the usage behavior of the user.
- Intended to automatically be provided at end of a sub-treatment action (e.g., after completion of a treatment stroke) or at end of treatment session in first and second usage mode.
- The FRA may depend on personalized data / information so that the FRA becomes "personalized feedback" (PFRA).
- The user may be allowed to deactivate individual elements of or the complete FRA to customize the first usage mode.

The term "a higher level of feedback and/or guidance and/or user interaction in the second usage mode" may thus be understood to mean that in the second usage mode General Guidance is given but not in the first usage mode and/or that Usage Guidance is given in the second usage mode but not in the first usage mode and/or that Interactive Usage Guidance is given in the second usage mode but not in the first usage mode and/or that Personalized Usage Guidance is given in the second usage mode but not in the first usage mode. This term may also be understood that each of the above listed guidance or feedback or interaction fields General Guidance, Usage Guidance, Interactive Usage Guidance, Personalized Usage Guidance, Feedback on Usage, Feedback on Results Achieved comprises one or several items or elements of individual guidance or feedback or interaction and that the second usage mode comprises more items or elements than the first usage mode. Such item or element may be one of the following examples (the skilled person will easily identify many more items or elements and this list is just given for getting the gist of the term): guidance on the use of a user-operable input element such as an on/off button or a trigger button, general guidance on the intended treatment and/or it medical and/or physical basics, guidance of skin features to be treated, guidance on safety features, guidance and/or interactive guidance and/or interactive guidance on correct usage of the skin treatment device including at least one of speed, number of light flash emission, distance between light flash emissions (both temporal and distance-wise), feedback and/or personalized feedback on the performance achieved during a treatment session, feedback and/or personalized feedback on the performance achieved during a portion of the treatment session such as a stroke done with the skin treatment device etc.

A skin treatment device or a skin treatment system in accordance with the present description comprises a user interface that includes at least one indicator element for providing user-perceivable information. The user interface may include at least one user operable input element provided on the skin treatment device. In a skin treatment system, the user interface may alternatively be located on a separate interaction device, or it may be distributed over the skin treatment device and the separate interaction device. E.g., the user interface may allow a user to input information via at least one first user operable input element provided on the skin treatment device and via a second user operable input element provided on the separate interaction device. It shall be understood as a general concept that in case of a skin treatment system individual features such as the user interface may be partly located on the skin treatment device and partly located on the separate interaction device. The at least one indicator element may be one of an audible, visual or tactile feedback element, e.g., a loudspeaker, a buzzer, a vibrator, a light element or a display. In case of two or more indicator elements, those may be of the same type or may be of a different type. Similarly, a processor can be located on the skin treatment device and/or on the separate interaction device. A processor may also be split such that some processing power of the processor is located in the skin treatment device and some processing power is located in the separate interaction device. Processing power, i.e., parts of the processor may alternatively or additionally be located in the cloud, e.g., on a server computer.

The skin treatment device or the skin treatment system in accordance with the present application has a first usage mode and a second usage mode. A processor of the skin treatment device / the skin treatment system is then arranged, i.e., is programmed or hardwired, to provide a higher level of user feedback and/or user guidance and/or user interaction in the second usage mode via the at least one indicator element during a usage session or treatment session (of the skin treatment device) than in the first usage mode. Usage session and treatment session are herein used interchangeably. These terms include that end of the treatment session, when the user does not perform any treatment anymore but has a look at the FRA (PFRA) if such is provided. By default, the first usage mode may be a standard usage mode in which the skin treatment device / the skin treatment system provides guidance and/or feedback of a standard level, e.g., only feedback on results achieved (FRA above) may be provided during a treatment session. The first usage mode shall also include the possibility that no feedback is provided at all. The second usage mode is then a mode in which a higher level of guidance and/or feedback is provided. E.g., in the second usage mode some first-time user guidance may be provided such as a pre-defined level of GG, UG (IUG/PUG). As such, the second usage mode may by default be the start mode that is automatically chosen in case the skin treatment device / skin treatment system is switched on for the first time. As an example, some introductory videos may be started that, e.g., explain details about the treatment provided by the skin treatment device / the skin treatment system and/or details about the handling of the skin treatment device / the skin treatment system.

That the second usage mode is typically automatically started, shall not exclude that the user is allowed to deactivate the second usage mode prior to using the skin treatment device / the skin treatment system. This may be sensible in case the user is already experienced but has bought a new skin treatment device / skin treatment system. The skin treatment device / skin treatment system may be arranged to switch into the second usage mode in case the processor analyzes that the usage behavior of the user indicates a first-time user or unexperienced user.

The skin treatment device / the skin treatment system may comprise a user-operable input element for switching from the first usage mode to the second usage mode and/or from the second usage mode to the first usage mode. It shall not be excluded that the skin treatment device / skin treatment system comprises three or more pre-defined usage modes in which different levels of guidance and/or feedback and/or user interaction are provided. While the user may be allowed to define the level of guidance and/or feedback and/or user interaction by manually activating or deactivating various guidance and/or feedback and/or user interaction elements, the availability of a plurality of usage modes may simplify finding the right level wishful for the user.

In accordance with some aspects, the processor is arranged for monitoring at least one usage parameter during a treatment session and for switching the skin treatment device / the skin treatment system into the second usage mode in case a value of the monitored usage parameter is above or below a predetermined threshold value or lies outside of a predetermined value range. The monitored usage parameter may be the time between treatment actions such as triggering events for light pulses to be applied on the skin. A high temporal distance between trigger events or an unevenly distributed temporal distance sequence between trigger events may indicate that the user is unexperienced and may benefit from a higher level of guidance and/or feedback and/or user interaction. The monitored usage parameter may relate to at least one from the list including but not being limited to skin contact of a treatment head of the skin treatment device, speed of the treatment head relative to the skin, use of a trigger button of the skin treatment device for triggering a skin treatment, number of skin treatments automatically or manually triggered during the treatment session, and temporal distance or sequence of temporal distances between individual skin treatments during a treatment session. Skin contact may be derivable from one or several skin contact sensors. In case the user regularly tries to trigger a treatment action even though (complete) skin contact is not given, this may be an indication that the user may benefit from a respective higher level of guidance and/or feedback and/or user interaction. In case the speed of the treatment head with respect to the skin is varying during a single stroke or between strokes or between treatment sessions may be an indication that the user may benefit from a respective higher level of guidance and/or feedback and/or user interaction. Repeated operation of a trigger button for triggering a treatment action even though the skin treatment device does not indicate that it is ready for a next treatment action may as well be an indication that the user may benefit from a respective higher level of guidance and/or feedback and/or user interaction.

The skin treatment device or the skin treatment system may comprise a user interface that allows a user to input at least one user parameter such as personal data. The processor may then be arranged to provide personalized feedback and/or personalized guidance and/or personalized user interaction that depends on the at least one user parameter. Non-limiting examples of user parameters may include age, weight, gender, body mass-index, hair color, skin tone, experience level of using the skin treatment device or system, ethnicity etc.

The processor may be arranged to automatically start the skin treatment device or skin treatment system in the second usage mode in case that the skin treatment device or system is used for the first time or in case a new user logs into the skin treatment device or skin treatment system or in case the skin treatment device or skin treatment system has not been used for a longer period, e.g., 4 weeks or 2 months or longer. The processor may also be arranged to start the skin treatment device or system in the first usage mode in case that the skin treatment device or system was previously used in the second usage mode during a complete treatment session, i.e., until completion of a standard guidance and/or feedback and/or interaction content of the second usage mode.

The processor may also be arranged to inhibit the use of the skin treatment device or skin treatment system for skin treatment until a predetermined completion level is achieved in the second usage mode.

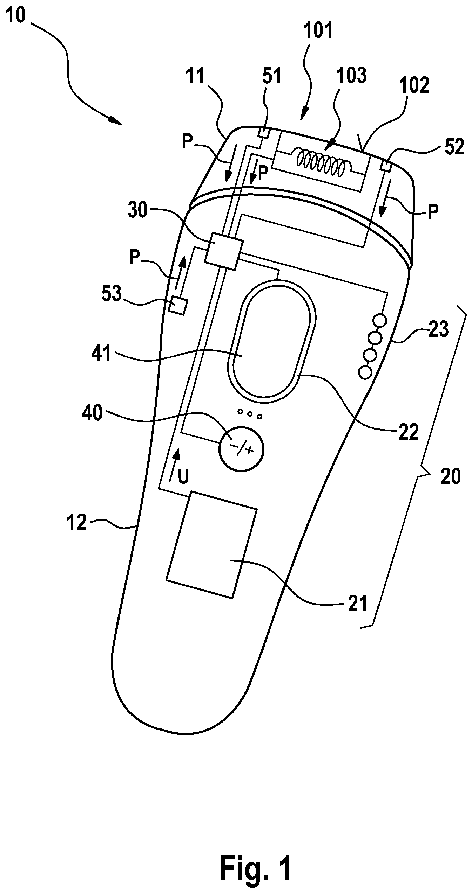

Fig. 1 is a schematic depiction of an example skin treatment device 10 realized as a light-based skin treatment device such as a light-based hair growth management device, commonly referred to as an IPL (Intense Pulsed Light) device. The skin treatment device 10 comprises a treatment head 11 and a handle 12. The treatment head 11 may itself be repeatedly attachable and again detachable from the handle 12 or the treatment head 11 may comprise a part (e.g., a cover part) that is repeatedly attachable and again detachable from the rest of the skin treatment device 10. Various elements of the skin treatment device 10 are schematically depicted. The treatment head 11 may comprise a treatment window 101 and a skin contact surface 102. The treatment head 11 may further comprise one or several sensors 51 and/or 52 that measure at least one usage parameter P. This shall not exclude that the skin treatment device alternatively or additionally comprises one several sensors 53 that determine one or several usage parameters, such as an inertial sensor to measure the speed, angulation, acceleration of the skin treatment device, a barometric sensor, a sensor that measures the magnetic field of the Earth etc. (see below). One of the sensors 51 may be realized as a skin contact sensor and the other sensor 52 may be realized as a skin tone sensor. In the shown example, the treatment head 11 also comprises a light source 103 such as a flash lamp that can be triggered to emit at least one flash onto skin.

The skin treatment device 10 comprises at least one indicator element 20, which indicator element 20 may be realized as a visual indicator element 21 or 23 such as a display 21, which may be touch sensitive, and/or an LED 23. As is shown in Fig. 1, a plurality of visual indicator elements 23 may be used. The indicator element 20 may alternatively or additionally comprise an audible and/or tactile indicator element, e.g., a buzzer or a vibrator 22, that here is realized as a frame around a button. The at least one indicator element 20 is arranged to provide user-perceivable information, by, e.g., switching on or off a light element such as an LED having a certain color, by flashing a light element in a certain pattern, by providing readable or visually recognizable information via a display, e.g., via displayed words and/or displayed pictures and/or videos, etc.

The skin treatment device 10 further comprises a processor 30 The processor 30 may be coupled with the at least one indicator element 20, with at least one sensor 51, 52, or 53 for measuring at least one usage parameter, where the skin treatment device may alternatively or additionally to the previously described sensors comprise at least one further sensor 53 that may be realized as an accelerometer, a gyroscope, a barometer, a magnetometer, a pressure sensor etc. The sensor 53 may, e.g., be realized as a muti-axis IMU (Inertia Measurement Unit) that may provide usage data P to the processor 30, the usage data then being motion data.

The skin treatment device 10 may comprise at least one user-operable input element 40 or 41. The at least one user-operable input element 40 may be realized as a button or switch for manually switching the skin treatment device from one of at least two usage modes to the other of the at least two usage modes, e.g., to switch the skin treatment device 10 from a first usage mode to a second usage mode. A second user-operable input element 41 may be realized as a switch or button for triggering a skin treatment, e.g., the emission of a light pulse.

As was previously discussed, the processor 30 may be arranged to provide at least a first usage mode and a second usage mode when using the skin treatment device 10, where the usage modes differ by the level of guidance and/or feedback and/or interaction as discussed before. E.g., the skin treatment device 10 may have a first usage mode that may be considered to be the "regular" usage mode, in which a low level of guidance and/or feedback and/or interaction is provided so that the user is essentially not disturbed when using the skin treatment device 10. The second usage mode may be considered a "new user" usage mode in which the level of guidance and/or feedback and/or interaction is provided is higher than in the first usage mode. An in-depth discussion of this has already been given in a previous section and it is here referred to this section. But in the context of this previous discussion, it is mentioned that the processor 30 may be arranged (e.g., hardwired or programmed) to utilize the at least one indicator element 21 to demand input from a user, which user input U may then be transferred to the processor 30 for further processing.

It shall be understood that the herein proposed "skin treatment device" shall not be limited to light-based skin treatment devices and that other devices such as massagers, brushes, epilation devices, electric shavers, wet shavers etc. shall be covered as well. The term "light-based skin treatment device" covers at least hair growth management devices or hair removal devices or beauty devices that, e.g., treat skin with light of one or various wavelengths to achieve a cosmetic or even therapeutic effect.

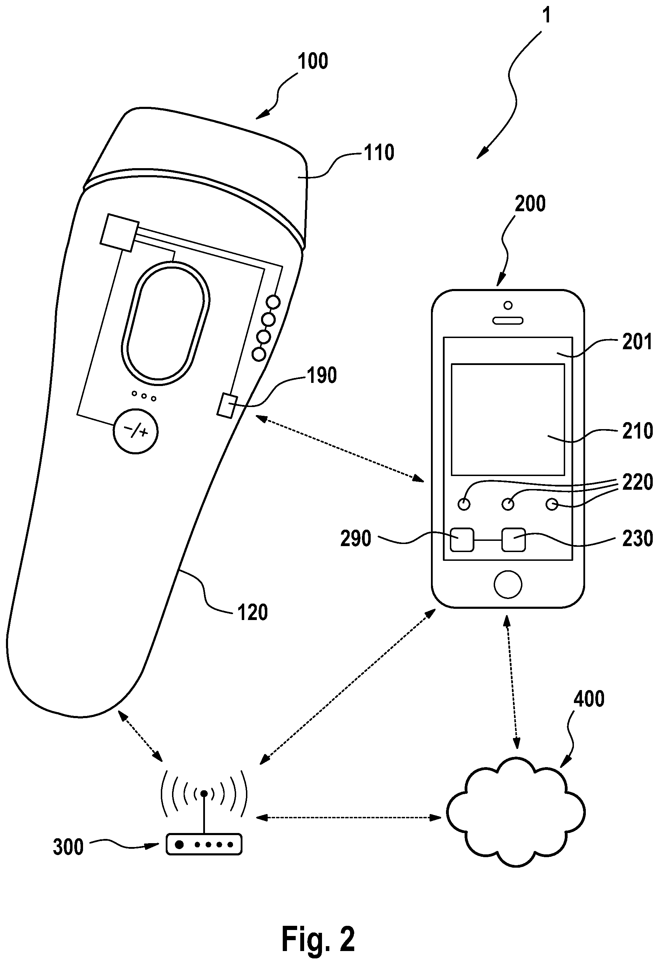

Fig. 2 is a schematic depiction of a skin treatment system 1 comprising a skin treatment device 100 and a separate interaction device 200. The skin treatment device 100 may comprise a treatment head 110 and a handle 120. The skin treatment device 100 may comprise all the features as discussed with reference to Fig. 1 and reference is made to the respective portion of the description. The skin treatment device 100 and the separate interaction device 200 are arranged for at least one-directional communication from the skin treatment device 100 to the separate interaction device 200. The communication may be a wired communication or a wireless communication. The communication may alternatively be bidirectional. In case of a wireless communication, the skin treatment device 100 may comprise a wireless communicator 190 and the separate interaction device 200 may comprise a wireless communicator 290 and the wireless communicators 190 and 290 are arranged for allowing at least one-directional or bidirectional communication. By means of the communication, the skin treatment device 100 can send usage data or performance data or the like to the separate interaction device 200.

The separate interaction device 200 may be realized by a mobile phone, smartphone, tablet or other small-sized computing device, e.g., as smart goggles or AR/VR glasses. In some embodiments, the separate interaction device 200 is realized by a proprietary device. The separate interaction device may have a processor 230 that may take over the processing done by the processor 30 in the examples discussed with respect to Fig. 1. This shall not exclude that the skin treatment device 100 has a processor that performs certain processing steps and the processor 230 may then perform additional processing steps. Without wanting to be limited, the separate interaction device 200 may comprise a display 201, specifically a touch sensitive display. The processor 230 may be arranged to provide guidance and/or feedback via at least one indicator element 210 and/or 220 that may be image and/or text areas on the display 21 and, depending on the needs, the processor 230 my be arranged to change the appearance and/or the content of what may be shown in the respective display areas realizing the at least one indicator element 210, 220. This shall not exclude that at least one indicator element is also present on the skin treatment device 100 as previously discussed. The information conveyed via the at least one indicator element 210, 220 on the separate interaction device 200 may be identical or different to the information conveyed via the discussed at least one indicator element on the skin treatment device 100. This shall not exclude that in a skin treatment system, all guidance and/or feedback and/or interaction is solely provided via the separate interaction device 200.

The skin treatment system 1 may be extended by further capabilities. E.g., the skin treatment device 100 may be arranged for at least one-directional communication with a router 300. Additionally or alternatively, the separate interaction device 200 may be arranged for at least one-directional communication with the router 300 and/or a cloud-based computing device 400. **In** the latter case, at least parts of the processing power of the processor 230 may be realized by the cloud-based computing device 400.

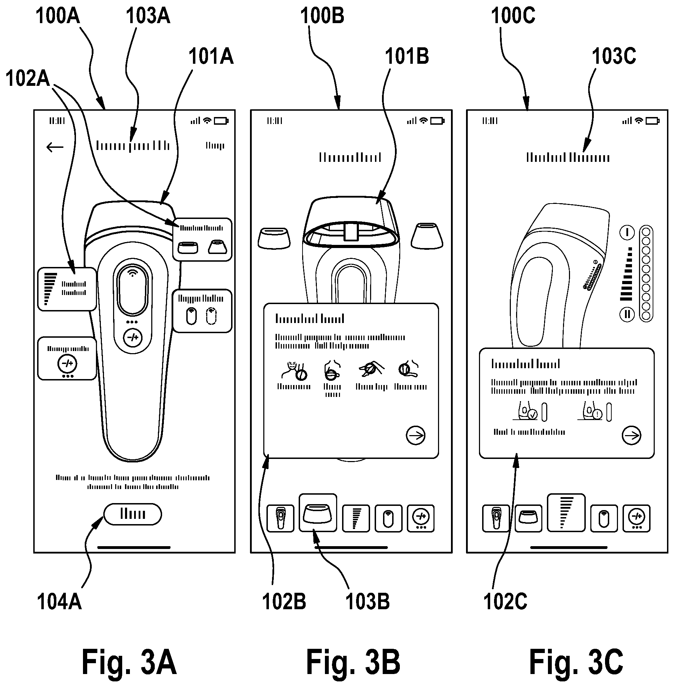

Figs. 3A - 3C, 4A - 4C, and 5 show information and/or input screens 100A - 100G that can be used in a teaching mode of a skin treatment device. The information and/or input screens are exemplary and may take any form and specifically are not to be understood as limiting, neither in their appearance nor in the information or data being shown or requested. The information and/or input screens 100A - 100G as shown may be part of a graphical user interface realized on a separate interaction device such as a smartphone.

Figs. 3A - 3C depict information and/or input screens 100A, 100B and 100C that shall support the user in understanding the functions and elements of a skin treatment device. The examples shown refers to an IPL device. These information and/or input screens 100A - 100C may be understood to provide usage guidance (UG) as discussed before. While the user, as will be explained, has the possibility to select and choose certain information and/or input screens, this shall not be understood as "interactive" as the latter sould imply a stage of user input based on a task or question provided.

On the information and/or input screen 100A an image 101A of a skin treatment device is shown as well as selectable information areas 102A that may be touched by the user to get more in depth information about certain functions or elements of the skin treatment device - see Figs. 3B and 3C. Additionally, the here shown information and/or input screen 100A has a general text area 103A that indicates the content of the current information and/or input screen and a general control area 104A that allows, e.g., to close the currently shown screen.

On the information and/or input screen 100B an image 101B of a skin treatment head is shown, which may be one of the elements that a user is able to select via one of the selectable information areas 102A on information and/or input screen 100A. Other skin treatment heads that can be chosen for using the skin treatment device are indicated to the left and right of the currently chosen skin treatment head and a user may select another skin treatment head by touching the respective image. The information and/or input screen 100B has an information area 102B on which information about the selected skin treatment head is shown and a selection area 103B via which a user may go to another information and/or input screen.

On the information and/or input screen 100C information about a function or non-hardware element of the skin treatment device is shown in an information area 102C. The function or non-hardware element that is explained is here indicated in a general text area 103C.

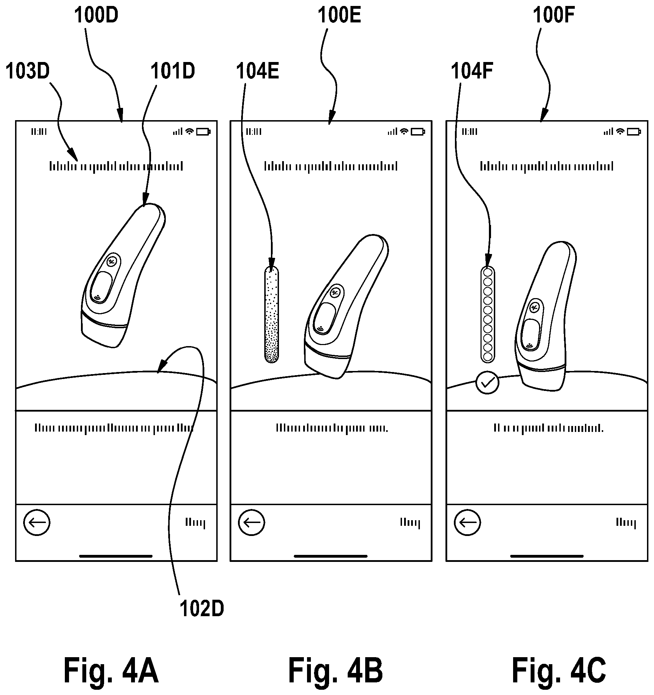

Figs. 4A to 4C depict a sequence of information and/or input screens 100D, 100E and 100F that shall teach the user the proper utilization of a skin treatment device with respect to skin contact. As an example, in an intense pulsed light (IPL) device, a high intensity light flash is repeatedly emitted onto the skin area to be treated. Such an intense light flash shall not reach the unprotected eye, and such devices typically only allow emission of a flash in case that close skin contact of an emission window is achieved so that the light is only emitted onto the skin. For an unexperienced user it might be frustrating in case the skin treatment device does not perform its treatment. Thus, the teaching mode may include information on the proper utilization so that the expected action indeed will occur.

On the information and/or input screen 100D in Fig. 4A, the user is told to move the skin treatment device onto the skin to establish good skin contact. Information and/or input screen 100D comprises an image 101D of a skin treatment device and a schematic depiction 102D of the skin, where both are at a distance to each other. A general text area 103D may be used to indicate the task the user is presented with, which may here be "Make a good skin contact".

Fig. 4B shows an information and/or input screen 100E that resembles the state in which the skin treatment device is not yet making good skin contact. Information and/or input screen 100E comprises a depiction of the skin treatment device and a depiction of the skin, where both are close together but not yet in contact. The information and/or input screen 100E may comprise a visual indicator area 104E to indicate whether or not good skin contact has been achieved, which here is not the case. This information may also be indicated by a visual indicator on the skin treatment device itself, which visual indicator may have a similar look as the visual indicator shown on the visual indicator area 104E.

In Fig. 4C an information and/or input screen 100F is shown that resembles the state in which the skin treatment device, i.e., a treatment head, has established good skin contact so that a light flash can be emitted through a light emission window, which may happen either automatically or by manually triggering it, e.g., with a trigger button provided at the device that can be operated by the user. The information and/or input screen 100F shows a depiction of the skin treatment device being in contact with the skin. A visual indicator area 104F shows a depiction of a visual indicator that may look as the respective visual indicator used at the skin treatment device itself.

The information and/or input screens 100D, 100E and 100F may be understood as an example of an interactive usage guidance (IUG) as the user is here presented with a task, namely to bring the skin treatment head into complete contact with the skin and the information and/or input screens 100E and 100F indicate the respective feedback that is given when the task is not yet achieved or is achieved, respectively.

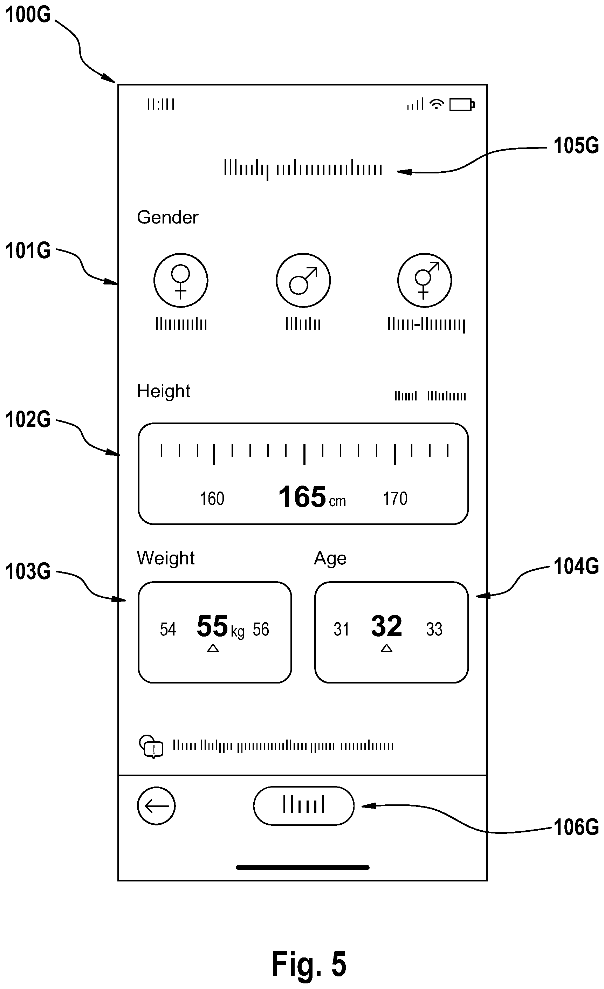

Fig. 5 depicts a potential interactive information and/or input screen 100G used to allow a user to input personal data such as the user's gender, body height, weight and age. This data is just exemplary. Other personal input data may involve skin dryness or density of liver spots. **In** the shown interactive information and/or input screen 100G, it shall be assumed that it is realized on a touch sensitive display. The information and/or input screen 100G comprises four input areas 101G, 102G, 103G, 104G, namely a gender input area 101G, a height input area 102G, a weight input area 103G and an age input area 104G. The gender input may then be provided by touching a selection button on the gender input area 101G indicating the correct gender such as "Female", "Male" or "Non-binary", to provide a non-limiting list of gender input examples. As is indicated in Fig. 5, the chosen gender may be visualized by highlighting the selection button - here it is "Female".

The height information can in the example shown be input in the height input area 102G via a slider bar. In Fig. 5, the chosen height is 165 cm. Similarly, the weight and the age can be input via respective slider bars in the weight input area 103G and in the age input area 104G, respectively. The chosen weight is here 55 kg and the chosen age is 32.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

## Claims

1. A skin treatment device, in particular a light-emitting hair growth management device such as an intense pulsed light device, comprising
a user interface including at least one indicator element for providing user perceivable information;
a first usage mode; and
at least a second usage mode;
a processor;
wherein the processor is arranged to provide a higher level of feedback and/or guidance and/or user interaction in the second usage mode via the at least one indicator element during a treatment session of the skin treatment device than in the first usage mode.

2. The skin treatment device in accordance with claim 1, a user-operable input element for switching the skin treatment device at least from one of the usage modes to another one of the usage modes.

3. The skin treatment device in accordance with claim 1 or claim 2, wherein the processor is arranged for monitoring at least one usage parameter during the usage session and for switching the skin treatment device into the second usage mode in case the monitored usage parameter lies above or below a predetermined threshold value or lies outside of a predetermined range.

4. The skin treatment device in accordance with one of claims 1 to 3, wherein the monitored usage parameter relates to at least one from a list including but not limited to skin contact of a treatment head of the skin treatment device, speed of the treatment head relative to the skin, use of a trigger button of the skin treatment device for triggering a skin treatment, number of skin treatments automatically or manually triggered during the treatment session, and temporal distance or temporal sequence of individual skin treatments during a treatment session.

5. The skin treatment device in accordance with one of claims 1 to 4, wherein the user interface is arranged to allow the user to enter at least one user parameter, and the processor is arranged to provide the user feedback and/or the user interaction in dependence on the at least one user parameter.

6. The skin treatment device in accordance with one of claims 1 to 5, wherein the processor is arranged to automatically start in the second usage mode in case the skin treatment device is used for the first time.

7. The skin treatment device in accordance with one of claims 1 to 6, wherein the processor is arranged to automatically start a usage session in the first mode in case the skin treatment device was previously used in the second usage mode during a complete usage session.

8. A skin treatment system comprising a skin treatment device, specifically a skin treatment device in accordance with one of claims 1 to 7, and a separate interaction device,
the separate interaction device and the skin treatment device being arranged for at least one-directional communication from the skin treatment device to the separate interaction device, in particular for bi-directional communication, further in particular wherein the communication is a wireless communication,
the separate interaction device comprising at least one indicator element for providing user-perceivable information,
the skin treatment system comprising a first usage mode, at least a second usage mode and a processor, and
wherein the processor is arranged to provide a higher level of user feedback and/or user interaction in the second usage mode via the at least one indicator element during a usage session of the skin treatment device than in the first usage mode.

9. The skin treatment system in accordance with claim 8, a user-operable input element for switching the skin treatment system at least from one of the usage modes to another one of the usage modes.

10. The skin treatment system in accordance with claim 8 or claim 9, wherein the processor is arranged for monitoring at least one usage parameter during the usage session and for switching the skin treatment system into the second usage mode in case the monitored usage parameter lies above or below a predetermined threshold value or lies outside of a predetermined range.

11. The skin treatment system in accordance with one of claims 8 to 10, wherein the monitored usage parameter relates to at least one from a list including skin contact of a treatment head of the skin treatment device, speed of the treatment head relative to the skin, use of a trigger button of the skin treatment device for triggering a skin treatment, number of skin treatments automatically or manually triggered during the treatment session, and temporal distance or temporal sequence of individual skin treatments during a treatment session.

12. The skin treatment system in accordance with one of claims 8 to 11, wherein the user interface is arranged to allow the user to enter at least one user parameter, and the processor is arranged to provide the user feedback and/or the user interaction in dependence on the at least one user parameter.

13. The skin treatment system in accordance with one of claims 8 to 12, wherein the processor is arranged to automatically start in the second mode in case the skin treatment system is used for the first time.

14. The skin treatment system in accordance with one of claims 8 to 13, wherein the processor is arranged to automatically start a usage session in the first mode in case the skin treatment system was previously used in the second mode during a complete usage session.

15. The skin treatment system in accordance with one of claims 8 to 14, wherein the processor is arranged to inhibit the use of the skin treatment device for skin treatment until the skin treatment system has achieved a predetermined completion level in the second usage mode.
